# EUROPÄISCHE PATENTANMELDUNG

(11) **EP 1 415 683 A1**
(43) Veröffentlichungstag der Anmeldung: **06.05.2004**
(21) Anmeldenummer: 03021367.2
(22) Anmeldetag: 22.09.2003
(51) Int. Cl.: A61N 5/06

(54) **Verfahren und Vorrichtung zum Bestrahlen des menschlichen Körpers mit UV-Strahlung**

(30) Priorität: 31.10.2002 DE 10251160
(71) Anmelder: Kern GmbH, 35759 Driedorf-Mademühlen (DE)
(72) Erfinder: Rommerskirchen, Jörg, 35756 Herborn (DE)
(74) Vertreter: Tappe, Hartmut

(57) **Zusammenfassung**

Die Erfindung betrifft ein Verfahren zum Betreiben einer Bestrahlungseinrichtung (1) zur Bestrahlung des menschlichen Körpers mit UV-Strahlung. Damit eine Manipulationsmöglichkeit der Bestrahlungsparameter durch den Benutzer ausgeschlossen ist und eine einfache Umrüstung bestehender Solarien möglich wird, sieht das Verfahren erfindungsgemäß folgende Schritte vor: a) Feststellen der Identität eines Benutzers (B) der Bestrahlungseinrichtung (1); b) Abfragen individueller, für die Bestrahlung relevanter Daten (D) des Benutzers (B); c) Eingeben der Daten (D) in eine zentrale Recheneinheit (2); d) Berechnen der für die Bestrahlung in der Bestrahlungseinrichtung (1) relevanten Parameter (P) in der zentralen Recheneinheit (2); e) Übertragen der berechneten Parameter (P) zur Bestrahlungseinrichtung (1), wobei der Benutzer (B) keine Einflussmöglichkeit auf die Parameter (P) hat; f) Aktivieren der Bestrahlungseinrichtung (1) zur Bestrahlung des Benutzers (B) mit den berechneten Parametern (P). Des weiteren betrifft die Erfindung eine Vorrichtung zur Bestrahlung.

## Beschreibung

Die Erfindung betrifft ein Verfahren zum Betreiben einer Bestrahlungseinrichtung zur Bestrahlung des menschlichen Körpers mit UV-Strahlung. Des weiteren betrifft die Erfindung eine Vorrichtung zum Bestrahlen.

Gattungsgemäße Verfahren und Vorrichtungen sind im Stand der Technik in mannigfaltiger Ausgestaltung bekannt. Beispielsweise offenbaren die DE 198 32 825 C2 und die DE 198 34 292 A1 Besonnungseinrichtungen, die insbesondere in Solarien zum Einsatz kommen.

Mit derartigen Bestrahlungseinrichtungen kann die Haut mit Ultraviolettstrahlung (UV-Strahlung) gebräunt werden, was zunächst aus Gründen des Erscheinungsbildes des Benutzers angestrebt werden kann. Darüber hinaus hat die zumindest gelegentliche Bestrahlung mit UV-Licht im bzw. vor dem Urlaub Sinn, um die Haut auf Phasen höherer natürlicher Bestrahlung vorzubereiten. Hierdurch können Nachteile vermieden werden, die bei ungewöhnlich höher Sonnenbestrahlung auftreten. Ferner ist eine künstliche Bestrahlung mit UV-Licht bei vielen medizinischen Indikationen vorteilhaft und fördert die Gesundheit.

In bekannten Besonnungseinrichtungen werden UV-Röhren mit unterschiedlicher Strahlungsintensität und Anzahl versehen. Einfluss auf die Bestrahlung kann auch durch die Anordnung der Bestrahlungsröhren relativ zum Körper des Benutzers erreicht werden. Aufgrund dessen ist die dem Körper des Benutzers zugeführte UV-Strahlung abhängig von der Art und Weise der Benutzung des Besonnungsgeräts und dessen Ausführungsform.

Bei bekannten Bräunungsanlagen wird die Besonnungsdauer oftmals lediglich durch Münzeinwurf festgelegt, so dass die individuellen Parameter der Bestrahlung nicht frei wählbar sein. Dies kann dazu führen, dass der Benutzer des Solariums, der eine schnelle Bräunung wünscht, die Bestrahlung gegebenenfalls durch zu häufige Benutzung des Solariums so stark erhöht, dass gesundheitlich Schäden auftreten.

Die menschliche Haut reagiert auf längere UV-Bestrahlung mit vermehrter Melaninbildung (Hyperpigmentierung) und mit einer Verdickung der Hornschicht. Dies dient dem natürlichen Lichtschutz. Melanine sind stickstoffhaltige, dunkle Farbstoffpigmente, die durch Oxidation, durch Ringschluss zum Indol und durch Polymerisation aus Dihydroxiphenylalanin entstehen. Bei der Haut geht die Melaninbildung speziell in den Basalzellen vor sich. Wenngleich es sich bei der Bildung von Melanin um eine natürliche Reaktion der Haut zu ihrem Schutz handelt, können gesundheitliche Schädigungen auftreten, wenn die individuelle, für die Bildung des Melanins benötigte Zeitdauer überschritten wird.

Dies kann von relativ geringfügigen Schädigungen (Hautrötung, Ödembildung) bis hin zum Hautkrebs führen.

Daher sind aus dem genannten Stand der Technik Besonnungseinrichtungen bekannt geworden, bei denen nach Erfassung der Identität eines Benutzers der Besonnungsanlage aus einer Datenbank die für ihn optimalen Besonnungsparameter ermittelt werden. Diese Daten werden dann beim Betrieb der Besonnungsanlage zugrunde gelegt, so dass eine übermäßige Strahlenbelastung mit UV-Licht vermieden wird.

Als nachteilig hat es sich dabei herausgestellt, dass die verfügbaren Anlagen nicht in der Lage sind, zuverlässig einen Schutz vor Manipulation der Daten durch den Benutzer sicherzustellen. Manche Besucher von Solarien wünschen nämlich trotz der damit verbundenen gesundheitlichen Gefährdungen eine sehr schnelle Bräunung und wollen eine zu lange Bestrahlung der Haut mit UV-Licht. Weiterhin hat es sich als problematisch herausgestellt, bestehende Solarien mit einer Anzahl von Besonnungsgeräten so auszustatten, dass die Bestrahlungsintensität in ökonomisch günstiger Weise in den einzelnen Bräunungsanlagen den individuellen Bedürfnissen der Benutzer angepasst werden kann. In der Regel ist es notwendig, dass hierfür eine komplett neue Ausstattung des Solariums mit Bräunungsgeräten erfolgt, um diese sämtlich so betreiben zu können, dass keine gesundheitlichen Schädigungen der Benutzer auftreten können.

Problematisch ist es in diesem Zusammenhang, dass neuere Strahlenschutzbestimmungen, die auch für den Betrieb von Solarien gelten, nur schwer bzw. nur mit erheblichem finanziellen Aufwand so ausgestattet werden können, dass der Betreiber des Solariums kein Risiko eingeht, dass er für einen Missbrauch seiner Bräunungsgeräte Haftungsansprüchen ausgesetzt ist.

Der Erfindung liegt daher die Aufgabe zugrunde, ein Verfahren zum Betreiben einer Bestrahlungseinrichtung und eine zugehörige Bestrahlungseinrichtung zu schaffen, die die vorbekannten Nachteile vermeiden hilft. Es soll insbesondere möglich sein, beim Betrieb der Bräunungsanlagen Manipulationsmöglichkeiten durch den Benutzer auszuschließen; weiterhin soll es möglich sein, auch bestehende Bräunungsanlagen in einer solchen Weise umzurüsten, dass ein zuverlässiger manipulationsfreier Betrieb sichergestellt ist. Dabei soll es dem Betreiber eines Solariums möglich sein, im Bedarfsfall den Nachweis dafür erbringen zu können, dass er die Benutzung seiner Bräunungsanlagen nur in einer solchen Weise zugelassen hat, dass gesetzliche Vorgaben hinsichtlich der Belastungsintensität der UV-Strahlung eingehalten wurden.

Die Lösung dieser Aufgabe durch die Erfindung erfolgt verfahrensgemäß durch die Abfolge der folgenden Schritte beim Betreiben einer Bestrahlungseinrichtung:
a) Feststellen der Identität eines Benutzers der Bestrahlungseinrichtung;
b) Abfragen individueller, für die Bestrahlung relevanter Daten des Benutzers;
c) Eingeben der Daten in eine zentrale Recheneinheit;
d) Berechnen der für die Bestrahlung in der Bestrahlungseinrichtung relevanten Parameter in der zentralen Recheneinheit;
e) Übertragen der berechneten Parameter zur Bestrahlungseinrichtung, wobei der Benutzer keine Einflussmöglichkeit auf die Parameter hat;
f) Aktivieren der Bestrahlungseinrichtung zur Bestrahlung des Benutzers mit den berechneten Parametern.

Wie insbesondere dem Schritt e) entnommen werden kann, erfolgt also die Übertragung der individuellen optimalen Parameter für die Bestrahlung mit UV-Licht zur Bestrahlungseinrichtung in einer solchen Weise, dass der Benutzer keine Einflussmöglichkeit auf die Parameter hat. Eine übermäßig hohe Bestrahlung wird dadurch zuverlässig vermieden.

Eine besondere Bedeutung kommt der Weiterbildung zu, dass die Übertragung der Parameter drahtlos erfolgt; hierbei ist insbesondere an eine Übertragung der Parameter durch Funk gedacht. Durch diese Maßnahme kann in besonders einfacher Weise ein bestehendes Solarium mit diversen Bräunungsgeräten ohne Manipulationsmöglichkeit durch den Benutzer umgerüstet werden.

Die Parameter umfassen dabei mit Vorteil die Bestrahlungszeit, die Intensität der Bestrahlung und/oder die Angabe der spektralen Gewichtung der Bestrahlung.

Ferner kann die Identität des Benutzers durch eine Identitätskarte, insbesondere eine Magnet- oder Chipkarte, durch Angabe einer individuellen Geheimnummer oder durch Erfassung einer körpereigenen Eigenschaft des Benutzers, insbesondere des Fingerabdrucks oder der Form der Iris, ermittelt werden.

Die individuellen, für die Bestrahlung relevanten Daten des Benutzers können Informationen über die Historie der Bestrahlung des Benutzers mit UV-Bestrahlung umfassen; ferner können sie Informationen über den Hauttyp des Benutzers umfassen.

Die Recheneinheit kann gemäß einer Weiterbildung Parameter für eine Anzahl Bestrahlungseinrichtungen, vorzugsweise für unterschiedliche Bestrahlungseinrichtungen, berechnen.

Die Vorrichtung zur Bestrahlung des menschlichen Körpers mit UV-Licht weist erfindungsgemäß die folgenden Elemente auf:
- mindestens eine Bestrahlungseinrichtung;
- Mittel zur Feststellung der Identität eines Benutzers der Bestrahlungseinrichtung;
- Speichermittel für die Speicherung individueller, für die Bestrahlung relevanter Daten des Benutzers;
- eine zentrale Recheneinheit zur Berechnung der für die Bestrahlung relevanten Parameter;
- Übertragungsmittel für die Übertragung der berechneten Parameter von der Recheneinheit zur Bestrahlungseinrichtung, wobei die Übertragungsmittel so ausgeführt sind, dass der Benutzer auf die Parameter keine Einflussmöglichkeit hat;
- Mittel zum Aktivieren der Bestrahlungseinrichtung für die Bestrahlung des Benutzers mit den berechneten Parametern.

Gemäß einer Weiterbildung sind die Übertragungsmittel als Mittel zum drahtlosen Übertragen der Parameter von der Recheneinheit zur der Bestrahlungseinrichtung ausgebildet. Die Übertragungsmittel können dabei als Funkübertragungsmittel ausgebildet sein.

Besonders bevorzugt kommen dabei zulassungsfreie Frequenzbänder zum Einsatz, weshalb vorgesehen werden kann, dass die Funkübertragungsmittel zum Betrieb auf einer Frequenz von 433 MHz oder 2,4 GHz ausgebildet sind.

Die Mittel zur Feststellung der Identität eines Benutzers können ein Magnetkartenleser oder ein Chipkartenleser sein. Gleichmaßen ist es möglich, dass diese Mittel ein Bilderkennungsgerät für den Fingerabdruck oder die Iris des Benutzers sind.

Die Recheneinheit kann über die Übertragungsmittel eine Anzahl Bestrahlungseinrichtungen mit Parametern versorgen; dabei kann vorgesehen sein, dass die Bestrahlungseinrichtungen nicht alle gleich ausgebildet sind.

Die Übertragungsmittel können mit Vorteil schließlich als nachrüstbare Elemente für die Bestrahlungseinrichtung bzw. Bestrahlungseinrichtungen ausgebildet sein.

Mit der vorgeschlagenen Verfahrensweise bzw. der Bestrahlungsvorrichtung ist eine Manipulationsmöglichkeit durch den Benutzer praktisch ausgeschlossen. Weiterhin können bestehende Solarien mit einem oder mehreren Bräunungsgeräten in sehr einfacher Weise umgerüstet werden, ohne hierfür hohe Investitionskosten in Kauf nehmen zu müssen. Über die zentrale Ansteuerung der Bestrahlungsvorrichtung bzw. der Bestrahlungsvorrichtungen über eine zentrale Recheneinheit ist es weiterhin in besonders einfacher Weise möglich, den individuellen Nachweis für jeden Benutzer darüber zu führen, welcher Benutzer welcher Strahleneinwirkung ausgesetzt war. Dies ist im Falle der Geltendmachung von Schadensersatzansprüchen gegen den Betreiber des Solariums von besonderer Bedeutung.

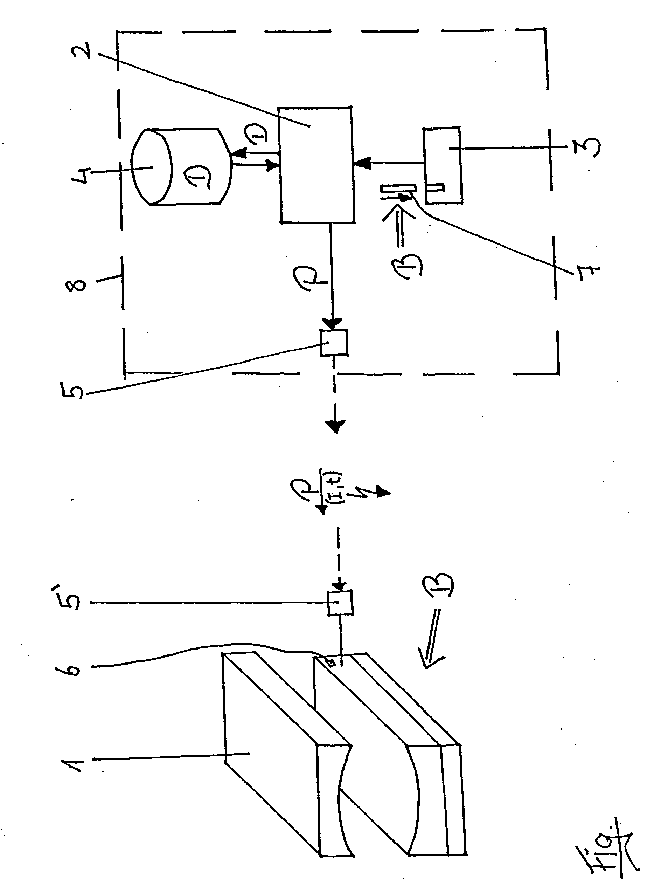

In der Zeichnung ist ein Ausführungsbeispiel der Erfindung dargestellt. Die einzige Figur zeigt schematisch ein Solarium mit einer Bestrahlungseinrichtung und einer zentralen Steuereinheit für diese.

Die Figur zeigt eine Bestrahlungseinrichtung 1 (Besonnungsgerät), in der ein Benutzer B einer Bestrahlung mit UV-Licht ausgesetzt werden kann, um den gewünschten Bräunungseffekt zu erreichen. Die für die Bestrahlung maßgeblichen Parameter P werden der Bestrahlungseinrichtung 1 von einer Recheneinheit 2 vorgegeben. Bei den Parametern P handelt es sich insbesondere um die Bestrahlungszeit t und die Intensität I der Strahlung.

Der Benutzer B begibt sich vor der Benutzung der Bestrahlungseinrichtung 1 zu einem Mittel 3 zur Feststellung seiner Identität. Bevorzugt kommt hierfür ein Magnetkartenleser zum Einsatz, in den der Benutzer B eine persönliche Magnetkarte 7 einführt.

Die Mittel 3 stehen mit der zentralen Recheneinheit 2 in Verbindung. An diese werden vom Mittel 3 diejenigen Daten geleitet, die den Benutzer B identifizieren. Die Recheneinheit 2 ihrerseits steht mit einem Speichermittel 4 in Form einer Datenbank in Verbindung, in dem Daten D hinterlegt sind. Bei diesen handelt es sich um individuelle Benutzerdaten, die über die Historie der Bestrahlung des Benutzers B Auskunft geben, also insbesondere über die Zeitpunkte, zu denen der Benutzer B mit UV-Strahlung vorgegebener Intensität I und vorgegebener Bestrahlungszeit t bestrahlt wurde.

Die Recheneinheit 2 ermittelt aufgrund der in ihr hinterlegten Algorithmen, die als solche bekannt und Stand der Technik sind, die für die anstehende Bestrahlung optimalen Parameter P, insbesondere also die bestmögliche Bestrahlungszeit t und gegebenenfalls Strahlungsintensität I.

Die ermittelten Parameter P werden an ein Übertragungsmittel 5 weitergeleitet (Sender), der über Funk mit einem Übertragungsmittel 5' (Empfänger) in Verbindung steht. Das Übertragungsmittel 5' (Empfänger) ist unmittelbar an der Bestrahlungseinrichtung 1 angeordnet, so dass hier dann die Parameter P vorliegen. An der Bestrahlungseinrichtung 1 ist ein Mittel 6 zum Aktivieren der Bestrahlungseinrichtung 1 angeordnet (EinSchalter), mit dem es dem Benutzer B, der sich in der Bestrahlungseinrichtung 1 befindet, lediglich möglich ist, die Bestrahlungseinrichtung 1 einzuschalten. Es ist ihm hingegen nicht möglich, die Parameter P und hier insbesondere die Bestrahlungszeit t zu verändern.

Die Übertragungsmittel 5, 5' basieren auf dem Prinzip einer seriellen Datenübertragung auf genehmigungsfreien Frequenzbändern. Bei dieser Funkübertragung kommt insbesondere das genehmigungsfreie 433 MHz- bzw. 2,4 GHz-Band zum Einsatz. Für die serielle Datenübertragung gilt die Norm RS 232 / RS 485.

Die einseitig ausgelegte Benutzerführung geht also von einer zentralen Steuereinheit 8 aus, der die Recheneinheit 2, die Mittel 3 zur Feststellung der Identität des Benutzers B, die Speichermittel 4 und die Übertragungsmittel 5 (Sender) zuzurechnen sind. Es besteht keine Manipulationsmöglichkeit für den Benutzer B, die Parameter P zu verändern. Die von der Recheneinheit 2 ermittelt Parameter P sind insofern bezüglich des Hauttyps des Benutzers, der Strahlungsintensität I der Bestrahlungseinrichtung 1 und der Bestrahlungshistorie des Benutzers B optimiert. Eine maximal mögliche bzw. sinnvolle Strahlendosis UV-Licht kann nicht überschritten werden.

In diesem Zusammenhang sei angemerkt, dass auch die bekannte allmähliche Alterung der Strahlungsquelle berücksichtigt werden kann, sofern der Recheneinheit 2 jeweils aktuelle Daten über den Alterungszustand der UV-Lampen zugeführt werden.

Die Aufnahme der sich teilweise verändernden nutzerspezifischen Daten wie Hauttyp oder schon vorhandene Strahlenbelastung in die Recheneinheit 2 bzw. in die Speichermittel 4 können in beliebiger Weise erfolgen, z. B. mittels einer Chipkarte oder durch Direkteingabe.

Es sein weiterhin noch auf die Möglichkeit hingewiesen, dass als Speichermittel 4 auch die Magnetkarte 7 selber genutzt werden kann; in diesem Falle sind also die individuellen Daten des Benutzers B sämtlich auf der Magnetkarte 7 (bzw. auf einem anderen Speichermedium) gespeichert. Dann kann ein zentrales Speichermittel 4 (Datenbank) entfallen.

Die Übertragungsmittel 5' (Empfänger), die an der Bestrahlungseinrichtung 1 angeordnet sind, können als nachrüstbare Module ausgebildet sein, die es in besonders einfacher Weise möglich machen, eine Umrüstung bestehender Solarien mit diversen Bestrahlungseinrichtungen zu bewerkstelligen.

Eine besonders bevorzugte Ausgestaltung des vorgeschlagenen Verfahrens sieht wie folgt aus:

Im Empfangsbereich eines Sonnenstudios wird die nur durch das Studiopersonal zugängliche Steuereinheit 8 installiert. Auf der Recheneinheit 2 sind die gerätespezifischen Daten (z. B. hauttypspezifische Bestrahlungszeiten) aller im Sonnenstudio vorhandenen Geräte gespeichert.

Die Mittel 3 zur Feststellung der Identität des Benutzers B sind als Magnetkartenleser ausgeführt. Der Benutzer B erhält nach einer Erstberatung seine individuellen Daten auf der Magnetkarte gespeichert, z. B. die letzte Bräunung mit zugehöriger Bestrahlungszeit.

Kommt der Benutzer erneut in das Sonnenstudio, werden seine Daten vom Mittel 3 gelesen. Im Abgleich mit den gerätespezifischen Daten der vom Benutzer B gewählten Bestrahlungseinrichtung 1 (Eingabe in die Recheneinheit 2 durch das Studiopersonal) werden die individuellen Bräunungsparameter (also namentlich die Bestrahlungszeit t in einer vorgegebenen Bestrahlungseinrichtung 1) ermittelt. Per Funkübertragung werden diese Parameter P zur Bestrahlungseinrichtung 1 übermittelt.

Der Benutzer B hat also keine nachträgliche Manipulationsmöglichkeit der Parameter P. Er kann lediglich nach dem Aufsuchen der Bestrahlungseinrichtung 1 diese durch die Mittel 6 aktivieren.

Die vorgeschlagene Vorrichtung hat den großen Vorteil, dass keine zusätzliche Verdrahtung des Sonnenstudios beim Nachrüsten mit der erfindungsgemäßen Vorrichtung erforderlich ist. Haftungsrechtliche Probleme werden durch die kontrollierte Anwendung der Bestrahlungseinrichtung 1 vermieden, d. h. der Betreiber eines Solariums wird in Bezug auf gesetzliche Vorgaben beim Betrieb der Bestrahlungseinrichtungen 1 unterstützt.

### Bezugszeichenliste

- 1: Bestrahlungseinrichtung
- 2: Recheneinheit
- 3: Mittel zur Feststellung der Identität
- 4: Speichermittel
- 5,5': Übertragungsmittel
- 6: Mittel zum Aktivieren
- 7: Magnetkarte
- 8: Steuereinheit

- B: Benutzer
- D: Daten
- P: Parameter
- t: Bestrahlungszeit
- I: Intensität

## Patentansprüche

1. Verfahren zum Betreiben einer Bestrahlungseinrichtung (1) zur Bestrahlung des menschlichen Körpers mit UV-Strahlung, das die Schritte aufweist:
a) Feststellen der Identität eines Benutzers (B) der Bestrahlungseinrichtung (1);
b) Abfragen individueller, für die Bestrahlung relevanter Daten (D) des Benutzers (B);
c) Eingeben der Daten (D) in eine zentrale Recheneinheit (2);
d) Berechnen der für die Bestrahlung in der Bestrahlungseinrichtung (1) relevanten Parameter (P) in der zentralen Recheneinheit (2);
e) Übertragen der berechneten Parameter (P) zur Bestrahlungseinrichtung (1), wobei der Benutzer (B) keine Einflussmöglichkeit auf die Parameter (P) hat;
f) Aktivieren der Bestrahlungseinrichtung (1) zur Bestrahlung des Benutzers (B) mit den berechneten Parametern (P).

2. Verfahren nach Anspruch 1,
**dadurch gekennzeichnet,**
**dass** die Übertragung der Parameter (P) drahtlos erfolgt.

3. Verfahren nach Anspruch 2,
**dadurch gekennzeichnet,**
**dass** die Übertragung der Parameter (P) per Funk erfolgt.

4. Verfahren nach einem der Ansprüche 1 bis 3,
**dadurch gekennzeichnet,**
**dass** die Parameter (P) die Bestrahlungszeit (t) umfassen.

5. Verfahren nach einem der Ansprüche 1 bis 4,
**dadurch gekennzeichnet,**
**dass** die Parameter (P) die Intensität (I) der Bestrahlung umfassen.

6. Verfahren nach einem der Ansprüche 1 bis 5,
**dadurch gekennzeichnet,**
**dass** die Parameter (P) die Angabe der spektralen Gewichtung der Bestrahlung umfassen.

7. Verfahren nach einem der Ansprüche 1 bis 6,
**dadurch gekennzeichnet,**
**dass** die Identität des Benutzers (B) durch eine Identitätskarte, insbesondere eine Magnet- oder Chipkarte, ermittelt wird.

8. Verfahren nach einem der Ansprüche 1 bis 7,
**dadurch gekennzeichnet,**
**dass** die Identität des Benutzers (B) durch Angabe einer individuellen Geheimnummer ermittelt wird.

9. Verfahren nach einem der Ansprüche 1 bis 6,
**dadurch gekennzeichnet,**
**dass** die Identität des Benutzers (B) durch Erfassung einer körpereigenen Eigenschaft des Benutzers (B), insbesondere des Fingerabdrucks oder der Form der Iris, ermittelt wird.

10. Verfahren nach einem der Ansprüche 1 bis 9,
**dadurch gekennzeichnet,**
**dass** die individuellen, für die Bestrahlung relevanten Daten (D) des Benutzers (B) Informationen über die Historie der Bestrahlung des Benutzers (B) mit UV-Bestrahlung umfassen.

11. Verfahren nach einem der Ansprüche 1 bis 10,
**dadurch gekennzeichnet,**
**dass** die individuellen, für die Bestrahlung relevanten Daten (D) des Benutzers (B) Informationen über den Hauttyp des Benutzers (B) umfassen.

12. Verfahren nach einem der Ansprüche 1 bis 11,
**dadurch gekennzeichnet,**
**dass** die Recheneinheit (2) Parameter (P) für eine Anzahl Bestrahlungseinrichtungen (1), vorzugsweise für unterschiedliche Bestrahlungseinrichtungen (1), berechnet.

13. Vorrichtung zur Bestrahlung des menschlichen Körpers mit UV-Strahlung, die aufweist:
- mindestens eine Bestrahlungseinrichtung (1);
- Mittel (3) zur Feststellung der Identität eines Benutzers (B) der Bestrahlungseinrichtung (1);
- Speichermittel (4) für die Speicherung individueller, für die Bestrahlung relevanter Daten (D) des Benutzers (B);
- eine zentrale Recheneinheit (2) zur Berechnung der für die Bestrahlung relevanten Parameter (P);
- Übertragungsmittel (5, 5') für die Übertragung der berechneten Parameter (P) von der Recheneinheit (2) zur Bestrahlungseinrichtung (1), wobei die Übertragungsmittel (5, 5') so ausgeführt sind, dass der Benutzer (B) auf die Parameter (P) keine Einflussmöglichkeit hat;
- Mittel (6) zum Aktivieren der Bestrahlungseinrichtung (1) für die Bestrahlung des Benutzers (B) mit den berechneten Parametern (P).

14. Vorrichtung nach Anspruch 13,
**dadurch gekennzeichnet,**
**dass** die Übertragungsmittel (5, 5') als Mittel zum drahtlosen Übertragen der Parameter (P) von der Recheneinheit (2) zu der Bestrahlungseinrichtung (1) ausgebildet sind.

15. Vorrichtung nach Anspruch 14,
**dadurch gekennzeichnet,**
**dass** die Übertragungsmittel (5, 5') als Funkübertragungsmittel ausgebildet sind.

16. Vorrichtung nach Anspruch 15,
**dadurch gekennzeichnet,**
**dass** die Funkübertragungsmittel (5, 5') zum Betrieb auf einer Frequenz von 433 MHz oder 2,4 GHz ausgebildet sind.

17. Vorrichtung nach einem der Ansprüche 13 bis 16,
**dadurch gekennzeichnet,**
**dass** die Mittel (3) zur Feststellung der Identität eines Benutzers (B) ein Magnetkartenleser oder ein Chipkartenleser sind.

18. Vorrichtung nach einem der Ansprüche 13 bis 16,
**dadurch gekennzeichnet,**
**dass** die Mittel (3) zur Feststellung der Identität eines Benutzers (B) ein Bilderkennungsgerät für den Fingerabdruck oder die Iris des Benutzers (B) sind.

19. Vorrichtung nach einem der Ansprüche 13 bis 18,
**dadurch gekennzeichnet,**
**dass** die Recheneinheit (2) über die Übertragungsmittel (5, 5') eine Anzahl Bestrahlungseinrichtungen (1) mit Parametern (P) versorgt.

20. Vorrichtung nach Anspruch 19,
**dadurch gekennzeichnet,**
**dass** die Bestrahlungseinrichtungen (1) nicht alle gleich ausgebildet sind.

21. Vorrichtung nach einem der Ansprüche 13 bis 20,
**dadurch gekennzeichnet,**
**dass** die Übertragungsmittel (5, 5') als nachrüstbare Elemente für die Bestrahlungseinrichtung bzw. Bestrahlungseinrichtungen (1) ausgebildet sind.
